Europäisches Patentamt

European Patent Office

Office européen des brevets

(11) Publication number: **0 140 498**
**B1**

# EUROPEAN PATENT SPECIFICATION

(45) Date of publication of patent specification: **31.05.89**

(51) Int. Cl.⁴: **A 61 K 7/16,** A 61 K 39/40

(21) Application number: **84305462.8**

(22) Date of filing: **10.08.84**

(54) Caries-preventive composition.

(30) Priority: **11.08.83 JP 146859/83**

(43) Date of publication of application:
**08.05.85 Bulletin 85/19**

(45) Publication of the grant of the patent:
**31.05.89 Bulletin 89/22**

(84) Designated Contracting States:
**AT BE CH DE FR GB IT LI NL**

(56) References cited:
**WO-A-82/04396**
**DE-A-2 757 290**
**GB-A-1 505 513**
**GB-A-2 008 948**
**GB-A-2 033 223**

**CHEMICAL ABSTRACTS, vol. 96, no. 11, March 15, 1982, Columbus, Ohio, USA, TOYO JOZO CO., LTD "Glucosyltransferase inhibitor M 5071", p. 467, abstract no. 84 106g**

**CHEMICAL ABSTRACTS, vol. 98, no. 3, January 7, 1983, Columbus, Ohio, USA, ENDO, AKIRA, "Physiologically active mutastein", p. 414, abstract no. 15 482f**

(73) Proprietor: **LION CORPORATION**
**No. 3-7, 1-chome Honjyo**
**Sumida-ku Tokyo (JP)**

(72) Inventor: **Miyahara, Tsuneo**
**No. 457, Yamanishi Ninomiya-machi**
**Naka-gun Kanagawa-ken (JP)**
Inventor: **Harada, Yoshihiro**
**No. 457, Yamanishi Ninomiya-machi**
**Naka-gun Kanagawa-ken (JP)**
Inventor: **Futakami, Katsuyuki**
**No. 34, Chigasaki Chigasaki-shi**
**Kanagawa-ken (JP)**

(74) Representative: **Ellis, John Clifford Holgate et al**
**MEWBURN ELLIS & CO. 2/3 Cursitor Street**
**London EC4A 1BQ (GB)**

Courier Press, Leamington Spa, England.

**Description**

This invention relates to a caries-preventive composition which, when applied to the mouth, can prevent dental caries by suppressing formation of dental plaque.

Dental plaque firmly adhering to the surface of teeth is composed of about 70% bacteria, about 20% polysaccharides produced by the bacteria and about 10% food remains. It is said that acids stored in dental plaque decalcify enamel, causing dental caries. Therefore, dental plaque is observed as a cause of dental caries.

Formation of dental plaque is accelerated due to the synthesis of polysaccharides from sucrose by oral bacteria, especially Streptococcus mutans. In more detail, Streptococcus mutans synthesizes adhesive polysaccharides such as dextran and mutan from sucrose through the production of GTF (glucosyltransferase, dextran-synthesizing enzyme). The thus synthesized polysaccharides incorporate Streptococcus mutans as well as other bacteria (viruses), forming dental plaque having a given bacterial bouquet. In addition, bacteria such as Streptococcus mutans produce acids by utilizing various kinds of sugar and the thus produced acids decalcify the surface of enamel by remaining in polysaccharides and bacterial walls.

Accordingly, it is desirable to decrease the number of Streptococcus mutans in the mouth and suppress the formation of dental plaque in order to prevent dental caries.

It is known both from GB—A—1,505,513 and WO—A—82/04396 that dental caries can be reduced or prevented by means of preparations containing antibodies to certain antigens derivable from S. mutans.

The present inventors studied antibodies which are amongst the antibodies to various antigens derived from Streptococcus mutans and inhibit the colonization of Streptococcus mutans in the mouth. As a result, the inventors found that antibodies contained in antiserum and milk obtained by immunizing mammals with Streptococcus mutans, its cell-wall fraction, fibrous substance fraction, glucosyltransferase fraction and protein antigen fraction have certain degrees of dental-plaque-formation suppressing effect. However, the effect was not necessarily sufficient and a higher effect of suppressing the formation of dental plaque was necessary.

An object of the present invention is to provide a caries-preventive composition having an excellent effect in preventing dental caries.

For the purpose of attaining the above object, the present inventors further conducted an intensive study, and, as a result, found that the combination of such an antibody and a fluorine compound, chlorhexidine or a chlorhexidine salt, a lytic enzyme, a bacteriocin, a glucosyltransferase inhibitor, a protease or a dextranase works effectively for the prevention of dental caries by causing a significantly increased dental-plaque-formation suppressing effect through the suppression of colonization of Streptococcus mutans.

It may be noted here that some of the aforementioned synergists for the aformentioned antibodies have themselves been used in toothpastes: thus, for example, DE—A—2757290 lists a number of fluorine compounds for use in the tooth-cleaning compositions of that invention, and comments that other substances known per se for inclusion in tooth-cleaning compositions, including proteases, dextranase and chlorhexidine may also be included. However, neither that document, nor any other of which we are aware, has disclosed the combination of the aforesaid materials with the antibodies referred to above.

Therefore, this invention provides a caries-preventive composition having a synergistic effect characterized by being composed of the combination of antibody obtained by immunizing a mammal with at least one antigen selected from Streptococcus mutans, its cell-wall fraction, fibrous substance fraction, glucosyltransferase fraction and protein antigen fraction with at least one synergist selected from fluorine compounds, chlorhexidine and its salts, lytic enzymes, bacteriocins, glucosyltransferase inhibitors, proteases and dextranases.

According to this invention, since the combination of said antibody and said synergist component exerts a synergistic effect on the inhibition of colonization of Streptococcus mutans in the mouth, the formation of dental plaque is efficiently suppressed, resulting in the effective prevention of dental caries.

In addition, since said antibody and said synergist component both are quite safe, the caries-preventive composition of this invention can be safely used.

The above and other objects, features, and advantages of this invention will be more fully understood by reading the following description.

The caries-preventive composition according to this invention is prepared by use of antibody contained in antiserum and/or milk obtained by immunizing a mammal with at least one antigen selected from the group consisting of Streptococcus mutans, its cell-wall fraction, fibrous substance fraction, glucosyltransferase (GTF) fraction and protein antigen fraction as described above. It should be noted that the fibrous substance means a pili-like or fimbriae fraction.

Streptococcus mutans used as an antigen may be prepared through well-known culture and pretreatment carried out by, for example, growing bacteria in external solution obtained by the dialysis of BHI medium before the thus grown bacteria are washed and subjected to formalin treatment. Streptococcus mutans separated from human mouth and belonging to the serotypes C, D, E, F and G may preferably be used, particularly one belonging to the serotype-C which is numerous in the human mouth. Such Streptococcus mutans includes NCTCI0449, Ingbritt OMZ70, JC-2, etc. and their mutant strains.

The cell-wall fraction of Streptococcus mutans may be prepared, for example, according to the method of Bleiweis et al. (J. Bacteriol., 88, 1198—1200, 1964) by subjecting Streptococcus mutans to crushing treatment in a Brown's cell crusher and glass beads of 0.17 to 0.18 mm diameter, then treating the thus obtained cell walls with trypsin to remove protein contaminating the cell walls, followed by washing the cell walls with distilled water before they are lyophilized. The fibrous (pili-like or fimbriae) substance fraction may be prepared, for example, according to the method of J. Van Hoate et al. (Arch. Oral. Bio., 16, 1131—1141, 1971) by culturing Streptococcus mutans in a medium obtained by the dialysis of BHI medium and containing 5% sucrose under an anaerobic condition, then centrifuging the culture medium to obtain a supernatant solution, then adding three times as much ethanol as the supernatant solution by volume, followed by collecting the precipitate of the thus obtained solution. As the fibrous substance fraction there may also be used a pili-like structure from the cell wall of Streptococcus mutans and its purified substance prepared by the ordinary cell wall extract method from the cultured bacteria, using solvents such as phosphate buffer containing 1M sodium chloride according to the method of Tsurumizu et al. (Japanese Journal of Bacteriology, 38, (1) 471, 1983). The GTF fraction may be prepared, for example, according to the method of Inoue et al. (Microbial Aspects of dental caries Vol. III, 665—682, 1976 [Information Retrieval Inc.]) using a solution prepared by the following method: after Streptococcus mutans is implanted and grown in a medium obtained by the dialysis of BHI medium, the bacterial bodies are removed by centifugation and the supernatant is saturated with ammonium sulfate at the level of 40%, followed by dialyzing the precipitate of the 40% ammonium sulfate fraction against 50 mm phosphate buffer solution and concentrating or diluting the obtained solution. The protein antigen fraction may be prepared, for example, according to the method of Lehner et al. (J. General Microbiology, 122, 217—225, 1981) by culturing Streptococcus mutans in a medium obtained by the dialysis of BHI medium, then centrifuging the culture medium to obtain a supernatant solution, followed by fractionation with 75% ammonium sulfate solution to collect the precipitate; the thus obtained precipitate is then subjected to DE—52 column chromatography under the existence of 6M urea, and the protein antigen fraction is dissolved in physiological saline, this being followed by dialyzing the thus obtained solution whereafter the dialyzed solution is subjected to gel filtration through Sepharose® CL6B.

The usual method may be adopted in immunizing mammals with said antigens. As mammals to be immunized, goats, sheep, horses, cows or rabbits may be used.

The antibody (protein fraction in the antiserum and the milk) may be separated from the antiserum and the milk according to the ordinary antibody purification method including the salting-out method, the gel-filtration method, ion-exchange chromatography or affinity chromatography, the salting-out method using ammonium sulfate being preferred. In the salting-out method, the antiserum or the milk is saturated with ammonium sulfate, preferably at the level of not more than 40%, to produce the precipitate, followed by dialyzing the precipitate against physiological saline to obtain the purified precipitate as the antibody. The preferred antibody is obtained from the equine antiserum and the bovine antiserum and milk.

In this invention, the antibody contained in the antiserum and milk obtained by immunizing the mammal with said antigen is blended into the composition. In this case, the antiserum and milk as well as the antibody separated and purified therefrom may be used. Each of these materials may be used alone or in a combination of two or more.

The caries-preventive composition according to this invention is prepared by the combination of said antibody and at least one synergist selected from the group consisting of fluorine compounds, chlorhexidine and its salts, lytic enzymes, bacteriocins, glucosyltransferase inhibitors, proteases and dextranases.

As fluorine compounds, sodium fluoride, potassium fluoride, lithium fluoride, ammonium fluoride, sodium monofluorophosphate, sodium hydrogen monofluorophosphate, potassium monofluorophosphate, ammonium monofluorophosphate, potassium hexafluorozirconate, and potassium hexafluorotitanate may be used. Also useful are cesium fluoride, nickel fluoride, zirconium fluoride, silver fluoride, hexylamine hydrofluoride, laurylamine hydrofluoride, cetylamine hydrofluoride, glycine hydrofluoride, lysine hydrofluoride or alanine hydrofluoride. Among them, monofluorophosphates such as sodium monofluorophosphate and potassium monofluorophosphate, alkali-metal fluorides such as sodium fluoride, potassium fluoride and ammonium fluoride, fluorides containing stannous tin such as stannous fluoride and stannous chloride fluoride may preferably be used. Especially, sodium monofluorophosphate, sodium fluoride and stannous fluoride are more preferably used.

As chlorhexidine salts, chlorhexidine hydrochloride or chlorhexidine gluconate can be used.

As lytic enzymes, those derived from Streptomyces griseus, Streptomyces diastatochromagenes, Streptomyces farinosus, Chalarpsis, Flavobacterium, Myxobacter, Staphyloccocus epidermidis, Micrococcus, Pseudomonas aetuginosa, Aeromanas, Stretomyces albus and Streptomyces globisporus can be used.

As bacteriocins, those derived from Enterobactor cloacae, Escherichia coli, Proteus mirabilis, Pseudomonas aeruginosa, Streptococcus mutans and Staphylococcus staphylolyticus can be used.

As GTF inhibitors, those derived from Arthrinum sp., Fusarinum sp., Macrophomina sp., Micromonospora sp., Gnomoniella sp., Nodulisporium sp., and Aspergillus sp., can be used, and more specifically, those described in Japanese Patent Application Laid-Open Nos. 56-103193, 57-28097, 57-98215 and 57-146587 can be used.

3

As proteases, those derived from Aspergillus sp., and Bacillus sp., can be used.

As dextranases, those derived from Chaetomium sp., Streptomyces sp., Bacillus sp. and Corynebacterium can be used.

In this invention, each of these synergist components may be used alone or in a combination of one or two.

The caries-preventive composition according to this invention can be prepared and used in various forms applicable to the mouth such as dentifrices (including toothpaste, toothpowder and liquid dentifrice), mouthwashes, dental pastes, gingival massage creams, gargle tablets, troches, chewing gums, ice-creams or whipped creams.

The antibody and the synergist component may be mixed in a given form. Alternatively, the antibody and the synergist component may be jointly used after they are prepared separately.

It is preferred that the quantity of said antibody administered is 0.0001 to 50 g/kg/day. As to the quantity of said synergist component administered, a quantity corresponding to 0.0001 to 1 g/kg/day fluorine for fluorine compounds, a quantity corresponding to 0.0001 to 1 g/kg/day chlorhexidine for chlorhexidine and its salts, a quantity of 0.0001 to 10 g/kg/day each for lytic enzymes, bacteriocins and glucosyltransferase inhibitors and a quantity of 0.0001 to 5 g/kg/day each for proteases and dextranases are preferably used. The blended amount of the antibody to the oral composition may be in the range of 0.0002 to 10%, preferably 0.002 to 5% by weight of the total weight of the composition. As to the blended amount of the synergist component in the composition, it is preferred that an amount corresponding to 0.0001 to 0.1 wt%, preferably 0.0001 to 0.001 wt% fluorine for fluorine compounds; an amount corresponding to 0.1 to 1000 ppm, preferably 10 to 100 ppm chlorhexidine for chlorhexidine and its salts; and an amount of 0.0001 to 10 wt%, preferably 0.001 to 5 wt% each for lytic enzymes, bacteriocins, glucosyltransferase inhibitors, proteases and dextranses may be blended to the composition.

The oral composition according to this invention may further include additional well-known ingredients depending on the type and form of a particular oral composition. Any desired known ingredients may be mixed with said antibody and synergist component.

In preparing dentifrice compositions, an abrasive may be blended generally in an amount of 5 to 95%, especially 15 to 60% by weight of the composition, including calcium secondary phosphate dihydrate, calcium secondary phosphate anhydrate, calcium primary phosphate, calcium tertiary phosphate, calcium carbonate, calcium pyrophosphate, insoluble sodium metaphosphate, magnesium tertiary phosphate, magnesium carbonate, calcium sulfate, titanium dioxide or resins.

In preparing paste-like compositions, typically toothpastes, a binder may be blended generally in an amount of 0.3 to 5% by weight, including sodium carboxymethyl cellulose, methyl cellulose, sodium carboxymethyl hydroxyethyl cellulose, hydroxyethyl cellulose, gum arabic, tragacanth gum, karaya gum, plyvinylalcohol, sodium polyacrylate, carboxyvinyl polymer or polyvinyl pyrrolidone.

In preparing paste-like and liquid oral compositions, typically toothpastes and mouthwashes, a humectant may be blended generally in an amount of 10 to 70% by weight, including polyethylene glycol, ethylene glycol, sorbitol, glycerol, propylene glycol, 1,3-butylene glycol, xylitol, maltitol or lactitol.

In addition to the above ingredients, a surface active agent including water soluble salts of alkyl sulfate having 8 to 18 carbon atoms such as sodium laurate and sodium myristate, sodium salts of higher fatty acids, water-soluble salts of sulfonated monoglycerides of higher fatty acids having 10 to 18 carbon atoms in the fatty acid group such as sodium lauryl monoglyceride sulfonate and sodium coconut monoglyceride sulfonate, sodium monoglyceride monosulfates of higher fatty acids, olefin sulfonates, paraffin sulfonates, sodium N-methyl-N-palmitoyl touride, sodium N-lauroyl sarcosinate, sodium N-lauroyl-β-alanine, stearyl monoglyceride, sucrose fatty acids esters having 12 to 18 carbon atoms in the fatty acid group such as sucrose monolaurate and dilaurate, lactose fatty acid esters, lactitol fatty acid esters, maltitol fatty acid esters, stearic acid monoglyceride, polyoxyethylene sorbitan monolaurate, polyoxyethylene-hardened castor oil, condensates of sorbitan monostearate with approximately 60 moles of ethylene glycol, condensates of ethylene oxide with propylene oxide, and their derivatives such as polyoxyethylene polyoxypropylene monolauryl ester, betaine or amino acid type amphoteric surfactants may be blended in an amount of 0 to 10%, preferably 0.1 to 5%, more preferably 1 to 2.5% by weight of the composition. A flavor such as an essential oil including peppermint oil and spearmint oil and a flavoring material including l-menthol, carvone, eugenol and anethole, a sweetener such as sodium saccharinate, stevioside, neohesperidyldihydrochalcone, glycyrrhizin, perillartine, p-methoxycinnamic aldehyde or a preservative may be blended in an effective amount.

In this invention, effective ingredients such as mutanase, sorbic acid, alexidine, hinokitiol, cetylpyridinium chloride, alkyl glycine, alkyldiaminoethyl glycinate, allantoin, ε-aminocaproic acid, tranexamic acid, azulene, vitamin E, a water soluble primary or secondary phosphate, a quaternary ammonium compound, sodium chloride and crude drugs may also be blended in an effective amount.

Other types of compositions may also be prepared by selecting any desired ingredients as usual and mixing them by a conventional procedure.

Examples of the other ingredients for various types or forms of the composition are shown in the following Examples.

Paste-like and liquid oral compositions may generally have a pH ranging from 5 to 10, but not limited thereto.

The cell-wall fraction of Streptococcus mutans may be prepared, for example, according to the method of Bleiweis et al. (J. Bacteriol., 88, 1198—1200, 1964) by subjecting Streptococcus mutans to crushing treatment in a Brown's cell crusher and glass beads of 0.17 to 0.18 mm diameter, then treating the thus obtained cell walls with trypsin to remove protein contaminating the cell walls, followed by washing the cell walls with distilled water before they are lyophilized. The fibrous (pili-like or fimbriae) substance fraction may be prepared, for example, according to the method of J. Van Hoate et al. (Arch. Oral. Bio., 16, 1131—1141, 1971) by culturing Streptococcus mutans in a medium obtained by the dialysis of BHI medium and containing 5% sucrose under an anaerobic condition, then centrifuging the culture medium to obtain a supernatant solution, then adding three times as much ethanol as the supernatant solution by volume, followed by collecting the precipitate of the thus obtained solution. As the fibrous substance fraction there may also be used a pili-like structure from the cell wall of Streptococcus mutans and its purified substance prepared by the ordinary cell wall extract method from the cultured bacteria, using solvents such as phosphate buffer containing 1M sodium chloride according to the method of Tsurumizu et al. (Japanese Journal of Bacteriology, 38, (1) 471, 1983). The GTF fraction may be prepared, for example, according to the method of Inoue et al. (Microbial Aspects of dental caries Vol. III, 665—682, 1976 [Information Retrieval Inc.]) using a solution prepared by the following method: after Streptococcus mutans is implanted and grown in a medium obtained by the dialysis of BHI medium, the bacterial bodies are removed by centifugation and the supernatant is saturated with ammonium sulfate at the level of 40%, followed by dialyzing the precipitate of the 40% ammonium sulfate fraction against 50 mm phosphate buffer solution and concentrating or diluting the obtained solution. The protein antigen fraction may be prepared, for example, according to the method of Lehner et al. (J. General Microbiology, 122, 217—225, 1981) by culturing Streptococcus mutans in a medium obtained by the dialysis of BHI medium, then centrifuging the culture medium to obtain a supernatant solution, followed by fractionation with 75% ammonium sulfate solution to collect the precipitate; the thus obtained precipitate is then subjected to DE—52 column chromatography under the existence of 6M urea, and the protein antigen fraction is dissolved in physiological saline, this being followed by dialyzing the thus obtained solution whereafter the dialyzed solution is subjected to gel filtration through Sepharose® CL6B.

The usual method may be adopted in immunizing mammals with said antigens. As mammals to be immunized, goats, sheep, horses, cows or rabbits may be used.

The antibody (protein fraction in the antiserum and the milk) may be separated from the antiserum and the milk according to the ordinary antibody purification method including the salting-out method, the gel-filtration method, ion-exchange chromatography or affinity chromatography, the salting-out method using ammonium sulfate being preferred. In the salting-out method, the antiserum or the milk is saturated with ammonium sulfate, preferably at the level of not more than 40%, to produce the precipitate, followed by dialyzing the precipitate against physiological saline to obtain the purified precipitate as the antibody. The preferred antibody is obtained from the equine antiserum and the bovine antiserum and milk.

In this invention, the antibody contained in the antiserum and milk obtained by immunizing the mammal with said antigen is blended into the composition. In this case, the antiserum and milk as well as the antibody separated and purified therefrom may be used. Each of these materials may be used alone or in a combination of two or more.

The caries-preventive composition according to this invention is prepared by the combination of said antibody and at least one synergist selected from the group consisting of fluorine compounds, chlorhexidine and its salts, lytic enzymes, bacteriocins, glucosyltransferase inhibitors, proteases and dextranases.

As fluorine compounds, sodium fluoride, potassium fluoride, lithium fluoride, ammonium fluoride, sodium monofluorophosphate, sodium hydrogen monofluorophosphate, potassium monofluorophosphate, ammonium monofluorophosphate, potassium hexafluorozirconate, and potassium hexafluorotitanate may be used. Also useful are cesium fluoride, nickel fluoride, zirconium fluoride, silver fluoride, hexylamine hydrofluoride, laurylamine hydrofluoride, cetylamine hydrofluoride, glycine hydrofluoride, lysine hydrofluoride or alanine hydrofluoride. Among them, monofluorophosphates such as sodium monofluorophosphate and potassium monofluorophosphate, alkali-metal fluorides such as sodium fluoride, potassium fluoride and ammonium fluoride, fluorides containing stannous tin such as stannous fluoride and stannous chloride fluoride may preferably be used. Especially, sodium monofluorophosphate, sodium fluoride and stannous fluoride are more preferably used.

As chlorhexidine salts, chlorhexidine hydrochloride or chlorhexidine gluconate can be used.

As lytic enzymes, those derived from Streptomyces griseus, Streptomyces diastatochromagenes, Streptomyces farinosus, Chalarpsis, Flavobacterium, Myxobacter, Staphyloccocus epidermidis, Micrococcus, Pseudomonas aetuginosa, Aeromanas, Stretomyces albus and Streptomyces globisporus can be used.

As bacteriocins, those derived from Enterobactor cloacae, Escherichia coli, Proteus mirabilis, Pseudomonas aeruginosa, Streptococcus mutans and Staphylococcus staphylolyticus can be used.

As GTF inhibitors, those derived from Arthrinum sp., Fusarinum sp., Macrophomina sp., Micromonospora sp., Gnomoniella sp., Nodulisporium sp., and Aspergillus sp., can be used, and more specifically, those described in Japanese Patent Application Laid-Open Nos. 56-103193, 57-28097, 57-98215 and 57-146587 can be used.

As proteases, those derived from Aspergillus sp., and Bacillus sp., can be used.

As dextranases, those derived from Chaetomium sp., Streptomyces sp., Bacillus sp. and Corynebacterium can be used.

In this invention, each of these synergist components may be used alone or in a combination of one or two.

The caries-preventive composition according to this invention can be prepared and used in various forms applicable to the mouth such as dentifrices (including toothpaste, toothpowder and liquid dentifrice), mouthwashes, dental pastes, gingival massage creams, gargle tablets, troches, chewing gums, ice-creams or whipped creams.

The antibody and the synergist component may be mixed in a given form. Alternatively, the antibody and the synergist component may be jointly used after they are prepared separately.

It is preferred that the quantity of said antibody administered is 0.0001 to 50 g/kg/day. As to the quantity of said synergist component administered, a quantity corresponding to 0.0001 to 1 g/kg/day fluorine for fluorine compounds, a quantity corresponding to 0.0001 to 1 g/kg/day chlorhexidine for chlorhexidine and its salts, a quantity of 0.0001 to 10 g/kg/day each for lytic enzymes, bacteriocins and glucosyltransferase inhibitors and a quantity of 0.0001 to 5 g/kg/day each for proteases and dextranases are preferably used. The blended amount of the antibody to the oral composition may be in the range of 0.0002 to 10%, preferably 0.002 to 5% by weight of the total weight of the composition. As to the blended amount of the synergist component in the composition, it is preferred that an amount corresponding to 0.0001 to 0.1 wt%, preferably 0.0001 to 0.001 wt% fluorine for fluorine compounds; an amount corresponding to 0.1 to 1000 ppm, preferably 10 to 100 ppm chlorhexidine for chlorhexidine and its salts; and an amount of 0.0001 to 10 wt%, preferably 0.001 to 5 wt% each for lytic enzymes, bacteriocins, glucosyltransferase inhibitors, proteases and dextranses may be blended to the composition.

The oral composition according to this invention may further include additional well-known ingredients depending on the type and form of a particular oral composition. Any desired known ingredients may be mixed with said antibody and synergist component.

In preparing dentifrice compositions, an abrasive may be blended generally in an amount of 5 to 95%, especially 15 to 60% by weight of the composition, including calcium secondary phosphate dihydrate, calcium secondary phosphate anhydrate, calcium primary phosphate, calcium tertiary phosphate, calcium carbonate, calcium pyrophosphate, insoluble sodium metaphosphate, magnesium tertiary phosphate, magnesium carbonate, calcium sulfate, titanium dioxide or resins.

In preparing paste-like compositions, typically toothpastes, a binder may be blended generally in an amount of 0.3 to 5% by weight, including sodium carboxymethyl cellulose, methyl cellulose, sodium carboxymethyl hydroxyethyl cellulose, hydroxyethyl cellulose, gum arabic, tragacanth gum, karaya gum, plyvinylalcohol, sodium polyacrylate, carboxyvinyl polymer or polyvinyl pyrrolidone.

In preparing paste-like and liquid oral compositions, typically toothpastes and mouthwashes, a humectant may be blended generally in an amount of 10 to 70% by weight, including polyethylene glycol, ethylene glycol, sorbitol, glycerol, propylene glycol, 1,3-butylene glycol, xylitol, maltitol or lactitol.

In addition to the above ingredients, a surface active agent including water soluble salts of alkyl sulfate having 8 to 18 carbon atoms such as sodium laurate and sodium myristate, sodium salts of higher fatty acids, water-soluble salts of sulfonated monoglycerides of higher fatty acids having 10 to 18 carbon atoms in the fatty acid group such as sodium lauryl monoglyceride sulfonate and sodium coconut monoglyceride sulfonate, sodium monoglyceride monosulfates of higher fatty acids, olefin sulfonates, paraffin sulfonates, sodium N-methyl-N-palmitoyl touride, sodium N-lauroyl sarcosinate, sodium N-lauroyl-β-alanine, stearyl monoglyceride, sucrose fatty acids esters having 12 to 18 carbon atoms in the fatty acid group such as sucrose monolaurate and dilaurate, lactose fatty acid esters, lactitol fatty acid esters, maltitol fatty acid esters, stearic acid monoglyceride, polyoxyethylene sorbitan monolaurate, polyoxyethylene-hardened castor oil, condensates of sorbitan monostearate with approximately 60 moles of ethylene glycol, condensates of ethylene oxide with propylene oxide, and their derivatives such as polyoxyethylene polyoxypropylene monolauryl ester, betaine or amino acid type amphoteric surfactants may be blended in an amount of 0 to 10%, preferably 0.1 to 5%, more preferably 1 to 2.5% by weight of the composition. A flavor such as an essential oil including peppermint oil and spearmint oil and a flavoring material including l-menthol, carvone, eugenol and anethole, a sweetener such as sodium saccharinate, stevioside, neohesperidyldihydrochalcone, glycyrrhizin, perillartine, p-methoxycinnamic aldehyde or a preservative may be blended in an effective amount.

In this invention, effective ingredients such as mutanase, sorbic acid, alexidine, hinokitiol, cetylpyridinium chloride, alkyl glycine, alkyldiaminoethyl glycinate, allantoin, ε-aminocaproic acid, tranexamic acid, azulene, vitamin E, a water soluble primary or secondary phosphate, a quaternary ammonium compound, sodium chloride and crude drugs may also be blended in an effective amount.

Other types of compositions may also be prepared by selecting any desired ingredients as usual and mixing them by a conventional procedure.

Examples of the other ingredients for various types or forms of the composition are shown in the following Examples.

Paste-like and liquid oral compositions may generally have a pH ranging from 5 to 10, but not limited thereto.

EP 0 140 498 B1

The caries-preventive composition according to this invention, owing to the combination of said antibody and said synergist component, can efficiently suppress the formation of plaque caused by Streptococcus mutans, thereby excellently preventing the formation of dental caries.

Examples of this invention will be given in the following to illustrate this invention.

## Example 1

Antisera and mother's milks were obtained by using the following antigens according to the following method.

(1) Antigens

Streptococcus mutans NCTC10449

Bacteria grown in the external solution obtained by the dialysis of BHI medium, after being washed, were treated with formalin before being supplied for use.

Cell-wall fraction of Streptococcus mutans NCTC10449

The fraction prepared according to the method of Bleiweis et al. (J. Bacteriol., *88,* 1198—1200, 1964) was supplied for use.

Fibrous substance fraction of Streptococcus mutans NCTC10449

The fraction prepared according to the method of J. Van Hoate et al. (Arch. Oral. Bio., *16,* 1131—1141, 1971) and Tsurumizu et al (Jap. J. Bacteriology, *38,* (1) 471, 1983) were supplied for use.

Glucosyltransferase fraction of Streptococcus mutans NCTC10449

The fraction prepared according to the method of Inoue et al. (Microbial Aspects of dental caries Vol. III, 665—682, 1976 [Information Retrieval Inc.]) was supplied for use.

Protein antigen fraction of Streptococcus mutans NCTC10449

The fraction prepared according to the method of Lehner et al. (J. General Microbiology, *122,* 217—225, 1981) was supplied for use.

(2) Preparation of Antiserum and Mother's Milk

Said antigen was mixed with Freund's complete adjuvant, and a pregnant goat, horse, cow or rabbit was immunized with the thus prepared mixture. After the animal was immunized three times with the mixture of said antigen and Freund's incomplete adjuvant before its delivery, the colostrum was collected after the delivery. As to the antiserum, after the animal was immunized four times in the same manner as above, the blood was collected and coagulated, and supernatant solution obtained by centifuging the coagulated blood was used as a sample.

An antibody is prepared by adding ammonium sulfate to the antiserum to saturate it at the level of 40%, separating the obtained precipitate by centrifugation, and dialyzing the precipitate against physiological saline, and the inner solution was used as a sample.

Next, the colonizing tests of streptococcus mutans in the mouth were conducted according to the following method by using said antiserum and mother's milk as well as a fluorine compound, a chlorhexidine salt, a lytic enzyme, a bacteriocin, GTF inhibitors, a protease and a dextranase used as synergist components.

(3) Colonization of Streptococus mutans in the Mouth

After male hamsters of five week old were divided into groups each consisting of five individuals, each hamster was inoculated with $1 \times 10^8$ bacteria of Streptococcus mutans of the NCTC10449 strain. From the day of the inoculation, drinking water containing the effective components (said antiserum or milk and the synergist component) was administered to each hamster. One week and four weeks after the start of the administration, the teeth of each hamster were rubbed with a cotton ball before it is immersed in a small amount of physiological saline to disperse bacteria homogeneously in it. After a given amount of the thus obtained solution was scattered on the BHI plate medium and the mitis salivalius plate medium, the number of whole bacteria and the number of the colonies of Streptococcus mutans were counted. The number of Streptococcus mutans was indicated by the number of Streptococcus mutans per 10,000 whole bacteria. The concentration of antiserum or mother's milk in the drinking water was adjusted to 0.025%. As to the concentration of the synergist component, it was adjusted to 0.05% for a fluorine compound (NaF), 0.005% for a chlorhexidine salt (chlorhexidine gluconate), 0.05% for a lytic enzyme, 0.01% each for a bacteriocin, a GTF inhibitor and a protease and 0.005% for a dextranase.

For comparison, the same experiments were conducted without jointly using antiserum or mother's milk and the synergist component by adding antiserum or mother's milk alone, by adding the synergist component alone and by adding none of antiserum, mother's milk and the synergist component (Control).

The results obtained by using the fluorine compound (NaF) as the synergist component are indicated in Table 1; those obtained by using chlorhexidine gluconate (CHX), in Table 2; those obtained by using the lytic enzyme, in Table 3; those obtained by using the bacteriocin, in Table 4; those obtained by using the GTF inhibitors, in Table 5; those obtained by using the protease, in Table 6; and those obtained by using the dextranase, in Table 7.

5

Table 1

| Samples Added | Number of S. mutans bacteria 1 week after | Number of S. mutans bacteria 4 weeks after |
|---|---|---|
| Control | 3890 | 4250 |
| Goat anti-whole-bacteria serum | 2178 | 1467 |
| "          + NaF | 1945 | 297 |
| Goat anti-GTF serum | 1828 | 1510 |
| "          + NaF | 1556 | 212 |
| Goat anti-whole-bacteria mother's milk | 1984 | 1382 |
| "          + NaF | 1945 | 170 |
| Goat anti-cell-wall serum | 1750 | 1340 |
| "          + NaF | 1750 | 255 |
| Goat anti-protein serum | 1984 | 1255 |
| "          + NaF | 1945 | 85 |
| Goat anti-fibrous-substance milk | 1945 | 1340 |
| "   ·      + NaF | 1711 | 127 |
| NaF alone | 3112 | 2040 |

# EP 0 140 498 B1

## Table 2

| Samples Added | Number of S. mutans bacteria 1 week after | Number of S. mutans bacteria 4 weeks after |
|---|---|---|
| Control | 3890 | 4250 |
| Antibody from equine anti-whole-bacteria serum | 2139 | 1425 |
| " + CHX | 2139 | 212 |
| Antibody from equine anti-GTF serum | 1789 | 1297 |
| " + CHX | 1634 | 340 |
| Equine anti-whole-bacteria mother's milk | 1945 | 1340 |
| " + CHX | 1984 | 170 |
| Equine anti-cell-wall serum | 2022 | 1425 |
| " + CHX | 1867 | 297 |
| Equine anti-protein serum | 2023 | 1425 |
| " + CHX | 1945 | 212 |
| Equine anti-fibrous-substance milk | 1867 | 1383 |
| " + CHX | 1134 | 85 |
| CHX alone | 3112 | 2975 |

Table 3

| Samples Added | | Number of S. mutans bacteria 1 week after | Number of S. mutans bacteria 4 weeks after |
|---|---|---|---|
| Control | | 3890 | 4250 |
| Bovine anti-whole-bacteria serum | | 2178 | 1425 |
| " | + Lytic enzyme | 1945 | 255 |
| Bovine anti-GTF serum | | 1828 | 1382 |
| " | + Lytic enzyme | 1556 | 340 |
| Bovine anti-whole-bacteria mother's milk | | 2023 | 1298 |
| " | + Lytic enzyme | 1945 | 127 |
| Antibody from bovine anti-cell-wall serum | | 2139 | 1255 |
| " | + Lytic enzyme | 1867 | 85 |
| Bovine anti-protein serum | | 1556 | 1085 |
| " | + Lytic enzyme | 1556 | 42 |
| Bovine anti-fibrous substance milk | | 2334 | 1298. |
| " | + Lytic enzyme | 1984 | 85 |
| Lytic enzyme alone | | 3112 | 3485 |

Note) As the lytic enzyme, one obtained from Streptomyces globisporus was used.

8

Table 4

| Samples Added | Number of S. mutans bacteria 1 week after | Number of S. mutans bacteria 4 weeks after |
|---|---|---|
| Control | 3890 | 4250 |
| Rabbit anti-whole-bacteria serum | 2178 | 1383 |
| " + Bacteriocin | 2100 | 298 |
| Rabbit anti-GTF serum | 1556 | 1467 |
| " + Bacteriocin | 1634 | 255 |
| Rabbit anti-whole-bacteria mother's milk | 1945 | 1510 |
| " + Bacteriocin | 1945 | 213 |
| Rabbit anti-cell-wall serum | 2023 | 1595 |
| " + Bacteriocin | 1634 | 170 |
| Rabbit anti-protein serum | 1945 | 1298 |
| " + Bacteriocin | 1751 | 128 |
| Rabbit anti-fibrous-substance milk | 2178 | 1383 |
| " + Bacteriocin | 1751 | 128 |
| Bacteriocin alone | 2723 | 3060 |

Note) As the bacteriocin, one obtained from Streptococcus L-1, microbial technology research laboratory trust number 3220, was used.

9

Table 5

| Samples Added | | Number of S. mutans bacteria 1 week after | Number of S. mutans bacteria 4 weeks after |
|---|---|---|---|
| Control | | 3890 | 4250 |
| Goat anti-whole-bacteria serum | | 2188 | 1425 |
| " | + GTF inhibitor A | 2100 | 212 |
| Goat anti-GTF serum | | 1867 | 1297 |
| " | + GTF inhibitor A | 1789 | 176 |
| Goat anti-whole-bacteria mother's milk | | 1945 | 1340 |
| " | + GTF inhibitor B | 1828 | 85 |
| Goat anti-cell-wall serum | | 2022 | 1425 |
| " | + GTF inhibitor A | 1945 | 340 |
| Goat anti-protein serum | | 1945 | 1297 |
| " | + GTF inhibitor C | 1906 | 255 |
| Goat anti-fibrous substance milk | | 2178 | 1383 |
| " | + GTF inhibitor A | 1751 | 128 |
| GTF inhibitor A alone | | 2723 | 3485 |
| GTF inhibitor B alone | | 3112 | 3400 |
| GTF inhibitor C alone | | 2995 | 3315 |

Note)    GTF inhibitor A was obtained from Aspergillus terreus;
         GTF inhibitor B, from Arthrinum sp. M 5071; and
         GTF inhibitor C, from Micromonospora sp. SF-2259.

Table 6

| Samples Added | Number of S. mutans bacteria 1 week after | Number of S. mutans bacteria 4 weeks after |
|---|---|---|
| Control | 3890 | 4250 |
| Equine anti-whole-bacteria serum | 2334 | 1297 |
| "          + Protease | 2022 | 85 |
| Equine anti-GTF serum | 1867 | 1340 |
| "          + Protease | 1789 | 85 |
| Equine anti-whole-bacteria mother's milk | 2022 | 1382 |
| "          + Protease | 1867 | 42 |
| Equine anti-cell-wall serum | 2178 | 1510 |
| "          + Protease | 2022 | 170 |
| Equine anti-protein serum | 1945 | 1552 |
| "          + Protease | 1711 | 42 |
| Equine anti-fibrous-substance milk | 2100 | 1340 |
| "          + Protease | 1634 | 170 |
| Protease alone | 3034 | 3655 |

Note)   The protease used is derived from Aspergillus sp.

EP 0 140 498 B1

Table 7

| Samples Added | Number of S. mutans bacteria 1 week after | Number of S. mutans bacteria 4 weeks after |
|---|---|---|
| Control | 3890 | 4250 |
| Bovine anti-whole-bacteria serum | 2334 | 1297 |
| "          + Dextra-nase | 2100 | 212 |
| Bovine anti-GTF serum | 2022 | 1510 |
| "          + Dextra-nase | 1789 | 212 |
| Bovine anti-whole-bacteria mother's milk | 2139 | 1552 |
| "          + Dextra-nase | 1945 | 340 |
| Bovine anti-cell-wall serum | 2139 | 1595 |
| "          + Dextra-nase | 1828 | 85 |
| Bovine anti-protein serum | 2022 | 1297 |
| "          + Dextra-nase | 1983 | 170 |
| Bovine anti-fibrous substance milk | 1867 | 1383 |
| "          + Dextra-nase | 1634 | 85 |
| Dextranase alone | 2022 | 1275 |

Note)    The dextranase used is derived from Chetomium sp.

12

From the results indicated in Tables 1 to 7, it is found that the combination of the antiserum or mother's milk and the synergist component according to this invention excellently suppresses the colonization of Streptococcus mutans.

Example 2
Toothpaste

| | |
|---|---|
| Calcium secondary phosphate dihydrate | 50.0% |
| Glycerol | 20.0 |
| Sodium carboxymethylcellulose | 1.0 |
| Sodium lauryl sulfate | 1.5 |
| Sodium lauroyl sarcosinate | 0.5 |
| Flavor | 1.0 |
| Sodium saccharinate | 0.1 |
| Water | Balance |
| | 100.0% |

The above components were blended with 0.1% or 0.2% antibody of goat whole-bacteria and 0.1% sodium fluoride, 0.01% chlorhexidine gluconate, 0.1% a lytic enzyme, 0.01% a bacteriocin, 0.001% a protease, 0.1% GTF inhibitor-A or 0.25% (3000 units/g) a dextranase.

Example 3
Toothpaste

| | |
|---|---|
| Calcium secondary phosphate | 50.0% |
| Sorbitol | 10.0 |
| Glycerol | 10.0 |
| Sodium carboxymethylcellulose | 1.0 |
| Sodium lauryl sulfate | 2.0 |
| Flavor | 1.0 |
| Sodium saccharinate | 0.1 |
| Ethanol | 2.0 |
| Mutanase | 0.1 |
| Water | Balance |
| | 100.0% |

The above components were blended with 0.1% bovine anti-cell-wall serum and 0.3% sodium monofluorophosphate, 0.1% chlorhexidine gluconate, 0.05% a lytic enzyme, 0.02% a bacteriocin, 0.001% a protease, 0.1% GTF inhibitor-C or 0.25% a dextranase.

13

Example 4
Toothpaste

| | |
|---|---|
| Calcium carbonate | 50.0% |
| Glycerol | 20.0 |
| Sodium carboxymethylcellulose | 1.5 |
| Sodium lauryl sulfate | 0.5 |
| Sucrose monolaurate | 2.0 |
| Flavor | 1.0 |
| Sodium saccharinate | 0.1 |
| Water | Balance |
| | 100.0% |

The above components were blended with 0.05% bovine anti-GTF mother's milk and 0.1% sodium fluoride, 0.005% chlorhexidine gluconate, 0.1% a lytic enzyme, 0.01% a bacteriocin, 0.001% a protease, 0.1% GTF inhibitor-B or 0.25% a dextranase.

Example 5
Toothpaste

| | |
|---|---|
| Calcium secondary phosphate dihydrate | 50.0% |
| Glycerol | 20.0 |
| Sodium carboxymethylcellulose | 2.0 |
| Sodium lauryl sulfate | 2.0 |
| Flavor | 1.0 |
| Sodium saccharinate | 0.1 |
| Water | Balance |
| | 100.0% |

The above components were blended with 0.1% equine anti-protein serum and 0.1% stannous fluoride, 0.01% chlorhexidine gluconate, 0.05% a lytic enzyme, 0.01% a bacteriocin, 0.001% a protease, 0.1% a GTF inhibitor or 0.25% a dextranase.

14

# EP 0 140 498 B1

### Example 6
### Toothpaste

| | |
|---|---|
| Calcium secondary phosphate dihydrate | 30.0% |
| Glycerol | 30.0 |
| Sorbitol | 20.0 |
| Sodium carboxymethylcellulose | 1.0 |
| Sodium lauryl sulfate | 2.0 |
| Flavor | 1.0 |
| Sodium saccharinate | 0.1 |
| Ethanol | 2.0 |
| Water | Balance |
| | 100.0% |

The above components were blended with 0.1% sheep anti-protein serum and 0.1% stannous fluoride, 0.01% chlorhexidine gluconate, 0.1% a lytic enzyme, 0.01% a bacteriocin, 0.0001% a protease, 0.1% GTF inhibitor-A or 0.17% (2000 units/g) a dextranase.

### Example 7
### Toothpowder

| | |
|---|---|
| Calcium secondary phosphate dihydrate | 50.0% |
| Calcium carbonate | 30.0 |
| Glycerol | 10.0 |
| α-olefin sulfonate | 1.0 |
| Flavor | 1.0 |
| Sodium saccharinate | 0.1 |
| Dextran | 0.5 |
| Water | Balance |
| | 100.0% |

The above components were blended with 0.1% sheep anti-fibrous-substance serum and 0.1% sodium monofluorophosphate and 0.1% sodium fluoride, 0.01% chlorhexidine gluconate, 0.05% a lytic enzyme, 0.001% a bacteriocin, 0.0001% a protease, 0.1% GTF inhibitor or 0.17% a dextranase.

15

# EP 0 140 498 B1

### Example 8
### Liquid Dentifrice

| | |
|---|---|
| Sodium polyacrylate | 50.0% |
| Glycerol | 30.0 |
| Flavor | 0.9 |
| Sodium saccharinate | 0.1 |
| Ethanol | 3.0 |
| Linolic acid | 0.05 |
| Water | Balance |
| | 100.0% |

The above components were blended with 0.01% or 0.02% goat anti-GTF mother's milk and 0.01% or 0.02% goat anti-protein mother's milk and 0.02% sodium fluoride, 0.05% chlorhexidine gluconate, 0.05% a lytic enzyme, 0.001% a bacteriocin, 0.002% a protease, 0.02% GTF inhibitor-A or 0.25% a dextranase.

### Example 9
### Mouthwash

| | |
|---|---|
| Ethanol | 20.0% |
| Flavor | 1.0 |
| Sodium saccharinate | 0.05 |
| Sucrose monolaurate | 0.3 |
| Water | Balance |
| | 100.0% |

The above components were blended with 0.1% goat anti-GTF serum and 0.1% sodium monofluorophosphate and 0.01% stannous fluoride, 0.01% chlorhexidine gluconate, 0.05% a lytic enzyme, 0.001% a bacteriocin, 0.01% a protease, 0.01% GTF inhibitor-B or 0.25% a dextranase.

### Example 10
### Mouthwash (tablet)

| | |
|---|---|
| Sodium hydrogencarbonate | 54.0% |
| Sodium secondary phosphate | 10.0 |
| Polyethylene glycol | 3.0 |
| Citric acid | 17.0 |
| Sodium sulfate (anhydrous) | 13.6 |
| Flavor | 2.0 |
| Oleic acid | 0.1 |
| | 100.0% |

16

# EP 0 140 498 B1

The above components were blended with 0.1% rabbit anti-GTF serum and 0.1% sodium monofluorophosphate and 0.05% sodium fluoride, 0.05% chlorhexidine gluconate, 0.05% a lytic enzyme, 0.01% a bacteriocin, 0.005% a protease, 0.05% GTF inhibitor-A or 0.25% a dextranase.

The tablet is used by dissolving 0.5 g of the tablet into 50 ml of water.

### Example 11
### Gingival Massage Cream

| | |
|---|---|
| White petrolatum | 8.0 |
| Propylene glycol | 4.0 |
| Stearyl alcohol | 8.0 |
| Polyethylene glycol 4000 | 25.0 |
| Polyethylene glycol 400 | 37.0 |
| Sucrose stearate | 0.5 |
| Water | Balance |
| | 100.0% |

The above components were blended with 0.5% bovine anti-fibrous-substance mother's milk and 0.5% sodium fluoride, 0.01% chlorhexidine gluconate, 0.05% a lytic enzyme, 0.01% a bacteriocin, 0.0% a protease, 0.5% GTF inhibitor-A or 0.25% a dextranase.

### Example 12
### Chewing Gum

| | |
|---|---|
| Gum base | 43.85% |
| Calcium carbonate | 2.0 |
| Starch syrup | 15.0 |
| Sugar | 30.0 |
| Sucrose palmitate | 1.0 |
| Fructose | 4.0 |
| Maltose | 3.0 |
| Flavor | 1.0 |
| | 100.0% |

The above components were blended with 0.1% bovine anti-whole-bacterial mother's milk and 0.1% stannous fluoride, 0.01% chlorhexidine gluconate, 0.1% a lytic enzyme, 0.01% a bacteriocin, 0.001% a protease, 0.1% GTF inhibitor-C or 0.25% a dextranase.

17

Example 13
Troche

| | |
|---|---|
| Gum arabic | 6.0% |
| Grape sugar | 75.0 |
| Flavor | 0.2 |
| l-menthol | 0.1 |
| Spearmint oil | 0.1 |
| Sodium ascorbate | 0.1 |
| Water | Balance |
| | 100.0% |

The above components were blended with 0.05% or 0.1% goat anti-protein serum and 0.05% sodium fluoride, 0.01% chlorhexidine gluconate, 0.05% a lytic enzyme, 0.01% a bacteriocin, 0.005% a protease, 0.1% GTF inhibitor-C or 0.25% a dextranase.

Example 14
Dental Paste

| | |
|---|---|
| Polyoxyethylene monostearate | 2.0% |
| Sorbitan monooleate | 2.0 |
| Cetyl alcohol | 2.0 |
| Palmityl alcohol | 3.0 |
| Propylene glycol | 15.0 |
| Carboxymethylcellulose | 5.0 |
| Saccharine | 0.2 |
| Peppermint oil | 0.5 |
| Spearmint oil | 0.5 |
| Lysozyme chloride | 5000 units/g |
| Water | Balance |
| | 100.0% |

The above components were blended with 0.05% or 0.1% equine anti-GTF serum and 0.05% sodium fluoride, 0.01% chlorhexidine hydrochloride, 0.05% a lytic enzyme, 0.01% a bacteriocin, 0.005% a protease, 0.1% GTF inhibitor-A or 0.25% a dextranase.

18

### Example 15
### Dental Paste

| | |
|---|---|
| Glyceryl monolaurate | 3.0% |
| Oleyl alcohol | 5.0 |
| Polyethylene glycol | 15.0 |
| White petrolatum | 3.0 |
| N-palmitoyl monosodium glutamate | 0.5 |
| Hydroxyethylcellulose | 5.0 |
| Tocopheryl acetate | 0.1 |
| Sodium saccharinate | 0.2 |
| Japanese peppermint oil | 0.7 |
| Cervone | 0.5 |
| Anethole | 0.3 |
| Eugenol | 0.1 |
| Water | Balance |
| | 100.0% |

The above components were blended with 0.025% or 0.05% rabbit anti-fibrous-substance serum and 0.05% sodium fluoride, 0.01% chlorhexidine hydrochloride, 0.05% a lytic enzyme, 0.001% a bacteriocin, 0.0025% a protease, 0.05% GTF inhibitor-B or 0.25% a dextranase.

### Example 16
### Ice-cream

| | |
|---|---|
| Cream (fat content, 50%) | 16.84% |
| Milk (fat content, 3.7%)* | 42.65 |
| Defatted evaporated milk | 24.24 |
| Sugar | 11.25 |
| Corn syrup | 4.65 |
| Stabilizer | 0.35 |
| | 100.0% |

*: Containing 0.5% bovine anti-fibrous-substance mother's milk

The above components were blended with 0.05% a lytic enzyme or 0.05% a bacteriocin.

19

# EP 0 140 498 B1

### Example 17
### Ice-cream

| | |
|---|---|
| Cream (fat content, 59%) | 16.84% |
| Milk (fat content, 3.7%)* | 42.65 |
| Defatted evaporated milk | 24.24 |
| Sugar | 11.25 |
| Corn syrup | 4.65 |
| Stabilizer | 0.35 |
| | 100.00% |

*: Containing 3% bovine anti-fibrous-substance mother's milk

The above components were blended with 0.001% a protease, 0.002% GTF inhibitor-C or 0.021% (250 units/g) a dextranase.

### Example 18
### Ice-cream

| | |
|---|---|
| Cream (fat content, 40%) | 31.54% |
| Milk (fat content, 3.7%)** | 37.16 |
| Defatted evaporated milk | 15.08 |
| Sugar | 11.25 |
| Corn syrup | 4.67 |
| Stabilizer | 0.30 |
| | 100.00% |

**: containing 5% bovine anti-protein mother's milk.

The above components were blended with 0.05% a lytic enzyme, 0.05% a bacteriocin, 0.001% a protease, 0.1% GTF inhibitor-A or 0.42% (5000 units/g) a dextranase.

## Claims

1. A caries-preventive composition having a synergistic effect comprising
an antibody obtained by immunizing a mammal with at least one antigen selected from Streptococcus mutans, its cell-wall fraction, fibrous substance fraction, glucosyltransferase fraction and protein antigen fraction, and
a synergist selected from the group consisting of fluorine compounds, chlorhexidine and its salts, lytic enzymes, bacteriocins, glucosyltransferase inhibitors, proteases and dextranases.

2. The composition as claimed in claim 1, wherein Streptococcus mutans is one belonging to the serotype C separated from human mouth.

3. The composition as claimed in claim 1 or 2, wherein the antibody is obtained from equine antiserum.

4. The composition as claimed in claim 1 or 2, wherein the antibody is obtained from bovine antiserum or milk.

5. The composition as claimed in any one of claims 1 to 4, wherein the antibody is prepared from the precipitate obtained by saturating the antiserum or milk with ammonium sulfate at the level of not more than 40%.

6. The composition as claimed in any preceding claim, wherein the blending amount of the antibody is in the range of 0.0002 to 10% by weight of the composition.

7. The composition as claimed in any preceding claim, wherein the synergist comprises a fluorine compound selected from monofluorophosphates, alkali-metal fluorides and fluorides containing stannous tin.

20

8. The composition as claimed in claim 7, wherein the fluorine compound is selected from sodium monofluorophosphate, sodium fluoride, and stannous fluoride.

9. The composition as claimed in any preceding claim, wherein the synergist comprises a fluorine compound and the blending amount of the fluorine compound is in the range of 0.0001 to 0.1% by weight of the composition as fluorine.

10. The composition as claimed in any one of claims 1 to 6, wherein the synergist comprises a chlorhexidine salt selected from chlorhexidine hydrochloride and chlorhexidine gluconate.

11. The composition as claimed in any one of claims 1 to 6 and 10 wherein the synergist comprises chlorhexidine or a chlorhexidine salt and the blending amount of chlorhexidine or its salt is in the range of 0.001 to 0.1% by weight of the composition.

12. The composition as claimed in any one of claims 1 to 6, including a synergist selected from lytic enzymes, bacteriocins, glucosyltransferase inhibitors, proteases and dextranases and the blending amount thereof is in the range of 0.001 to 10% by weight of the composition.

13. The composition as claimed in any preceding claim wherein the composition is prepared as a dentifrice, a mouthwash, an oral paste or a gingival massage cream.

14. The composition as claimed in any one of claims 10 to 12, wherein the composition is prepared as a troche or a chewing gum.

15. The composition as claimed in claim 12, wherein the composition is prepared as an ice cream.

**Patentansprüche**

1. Kariesverhütende Zusammensetzung mit synergistischer Wirkung umfassend einen Antikörper, der durch Immunisieren eines Säugetieres mit wenigstens einem Antigen erhalten wird, das aus Streptococcus mutans, seiner Zellwandfraktion, Fasersubstanzfraktion, Glucosyltransferasefraktion und Proteinantigenfraktion gewählt wird und
einen Synergisten, der aus der Gruppe erhalten wird, die aus Fluorverbindungen, Chlorhexidin und seinen Salzen, lytischen Enzymen, Bacteriocinen, Glucosyltransferaseinhibitoren, Proteasen und Dextranasen besteht.

2. Zusammensetzung nach Anspruch 1, worin der Streptococcus mutans ein solcher des Serotyps C ist, der aus dem menschlichen Mund isoliert wird.

3. Zusammensetzung nach Anspruch 1 oder 2, worin der Antikörper aus Pferde-Antiserum erhalten wird.

4. Zusammensetzung nach Anspruch 1 oder 2, worin der Antikörper aus Rinder-Antiserum oder -milch erhalten wird.

5. Zusammensetzung nach einem der Ansprüche 1 bis 4, worin der Antikörper aus dem Niederschlag hergestellt wird, der durch Sättigung des Antiserums oder der Milch mit Ammoniumsulfat in einem Ausmaß von nicht mehr als 40% erhalten wird.

6. Zusammensetzung nach einem der vorhergehenden Ansprüche, worin die Menge, in der der Antikörper zugemischt wird, im Bereich von 0,0002 bis 10 Gew.-% der Zusammensetzung liegt.

7. Zusammensetzung nach einem der vorhergehenden Ansprüche, worin der Synergist eine Fluorverbindung enthält, die aus Monofluorphosphaten, Alkalimetallfluoriden und Zinn (II) enthaltenden Fluoriden gewählt ist.

8. Zusammensetzung nach Anspruch 7, worin die Fluorverbindung aus Natriummonofluorphosphat, Natriumfluorid und Zinn(II)fluorid gewählt ist.

9. Zusammensetzung nach einem der vorhergehenden Ansprüche, worin der Synergist eine Fluorverbindung enthält und die zugemischte Menge an Fluorverbindung, ausgedrückt als Fluor, im Bereich von 0,0001 bis 0,1 Gew.-% der Zusammensetzung liegt.

10. Zusammensetzung nach einem der Ansprüche 1 bis 6, worin der Synergist ein Chlorhexidinsalz enthält, das aus Chlorhexidinhydrochlorid und Chlorhexidingluconat gewählt ist.

11. Zusammensetzung nach einem der Ansprüche 1 bis 6 und 10, worin der Synergist Chlorhexidin oder ein Chlorhexidinsalz enthält und die zugemischte Menge an Chlorhexidin oder seines Salzes im Bereich von 0,001 bis 0,1 Gew.-% der Zusammensetzung liegt.

12. Zusammensetzung nach einem der Ansprüche 1 bis 6, umfassend einen Synergisten, der aus lytischen Enzymen, Bacteriocinen, Glucosyltransferaseinhibitoren, Proteasen und Dextranasen gewählt ist und in einer Menge innerhalb eines Bereiches von 0,001 bis 10 Gew.-% der Zusammensetzung zugemischt wird.

13. Zusammensetzung nach einem der vorhergehenden Ansprüche, worin die Zusammensetzung als Zahnputzmittel, Mundwasser, orale Paste oder Zahnfleischmassagecreme zubereitet wird.

14. Zusammensetzung nach einem der Ansprüche 10 bis 12, worin die Zusammensetzung als Pastille oder Kaugummi zubereitet wird.

15. Zusammensetzung nach Anspruch 12, worin die Zusammensetzung als Eiscreme zubereitet wird.

**Revendications**

1. Composition pour éviter les caries dentaires ayant un effet synergique, comprenant:

— un anticorps obtenu par immunisation d'un mammifère avec au moins un antigène choisi parmi Streptococcus mutans, ses fraction de paroi cellulaire, fraction de substance fibreuse, fraction de glucosyltransférase et fraction d'antigène de protéine, et

— une substance synergique choisie dans le groupe constitué de composés fluorés, de la chlorhexidine et ses sels, d'enzymes lytiques, de bactériocines, d'inhibiteurs de glucosyltransférase, de protéases et de dextranases.

2. Composition selon la revendication 1, dans laquelle Streptococcus mutans est celle appartenant au sérotype C séparé de la bouche humaine.

3. Composition selon la revendication 1 ou 2, dans laquelle l'anticorps est obtenu à partir d'antisérum chevalin.

4. Composition selon la revendication 1 ou 2, dans laquelle l'anticorps est obtenu à partir d'antisérum ou de lait bovin.

5. Composition selon l'une quelconque des revendications 1 à 4, dans laquelle l'anticorps est préparé à partir du précipité obtenu en saturant l'antisérum ou le lait avec du sulfate d'ammonium à un taux inférieur ou égal à 40%.

6. Composition selon l'une des revendications précédentes, dans laquelle la quantité de mélange de l'anticorps est dans la plage de 0,0002 à 10% en poids de la composition.

7. Composition selon l'une quelconque des revendications précédentes, dans laquelle la substance synergique comprend un composé fluoré choisi parmi des monofluorophosphates, des fluorures de métaux alcalins et des fluorures contenant de l'étain stanneux.

8. Composition selon la revendication 7, dans laquelle le composé fluoré est choisi à partir du monofluorophosphate de sodium, du fluorure de sodium et du fluorure stanneux.

9. Composition selon l'une quelconque des revendications précédentes, dans laquelle la substance synergique comprend un composé fluoré et la quantité de mélange du composé fluoré est dans la plage de 0,0001 à 0,1% en poids de la composition en fluor.

10. Composition selon l'une quelconque des revendications 1 à 6, dans laquelle la substance synergique comprend un sel de chlorhexidine choisi parmi l'hydrochlorure de chlorhexidine et le gluconate de chlorhexidine.

11. Composition selon l'une quelconque des revendications 1 à 6 et 10, dans laquelle la substance synergique comprend la chlorhexidine ou un sel de chlorhexidine et la quantité de mélange de la chlorhexidine ou de son sel est dans la plage de 0,001 à 0,1% en poids de la composition.

12. Composition selon l'une quelconque des revendications 1 à 6, comportant une substance synergique choisie à partir d'enzymes lytiques, de bactériocines, d'inhibiteurs de glucosyltransférase, de protéases et de dextranases et la quantité de mélange de celle-ci est dans la plage de 0,001 à 10% en poids de la composition.

13. Composition selon l'une quelconque des revendications précédentes, dans laquelle la composition est préparée sous forme d'un dentifrice, d'un bain de bouche, d'une pâte orale ou d'une crème de massage gingivale.

14. Composition selon l'une quelconque des revendications 10 à 12, dans laquelle la composition est préparée sous forme d'une pastille ou d'un chewing-gum.

15. Composition selon la revendication 12, dans laquelle la composition est préparée sous forme d'une crème glacée.